# NEUE EUROPÄISCHE PATENTSCHRIFT

(11) **EP 1 230 939 B2**
(45) Veröffentlichungstag und Bekanntmachung des Hinweises auf die Entscheidung über den Einspruch: **19.01.2011**
(45) Hinweis auf die Patenterteilung: 04.10.2006
(21) Anmeldenummer: 01103327.1
(22) Anmeldetag: 13.02.2001
(51) Int. Cl.: A61L 27/36, A61L 27/38

(54) **Bioartifizielle, primär vaskularisierte Gewebematrix und bioartifizielles, primär vaskularisiertes Gewebe**
Bioartificial vascularised tissue matrix and bioartificial vascularised tissue
Matrice tissulaire bioartificielle vascularisée et tissu bioartificiel vascularisé

(43) Veröffentlichungstag der Anmeldung: 14.08.2002
(73) Patentinhaber: corLife GbR, 30625 Hannover (DE)
(72) Erfinder: Haverich, Axel, Prof., Dr., 30916 Isernhagen (DE); Mertsching, Heike, Dr., 31535 Neustadt (DE)
(74) Vertreter: Grünecker, Kinkeldey, Stockmair & Schwanhäusser Anwaltssozietät

(56) Entgegenhaltungen:
- EP-A2- 0 128 706
- WO-A-99/52356
- WO-A1-01/48153
- WO-A1-96/40889
- WO-A2-99/00152
- CA-A- 1 261 290
- DE-A1- 10 021 627
- DE-A1- 10 034 583
- US-A- 5 632 778
- US-A- 5 855 610
- US-A- 5 855 620
- BADER A. ET AL: 'ENGINEERING OF HUMAN VASCULAR AORTIC TISSUE BASED ON A XENOGENEIC STARTER MATRIX' TRANSPLANTATION Bd. 70, Nr. 1, 15 Juli 2000, USA, Seiten 7 - 14
- STEINHOFF G. ET AL: 'TISSUE ENGINEERING OF PULMONARY HEART VALVES ON ALLOGENIC ACELLULAR MATRIX CONDUITS In Vivo Restoration of Valve Tissue' CIRCULATION 102 ¬SUPPL.III| 07 November 2000, Seiten III-50 - III-55
- TEEBKEN O.E. ET AL: 'TISSUE ENGINEERING OF VASCULAR GRAFTS: HUMAN CELL SEEDING OF DECELLULARISED PORCINE MATRIX' EUR.J.VASC.ENDOVASC.SURG. Bd. 19, April 2000, HARCOURT PUBLISHERS LTD., Seiten 381 - 386
- HUYNH T. ET AL: 'REMODELING OF AN ACELLULAR COLLAGEN GRAFT INTO A PHYSIOLOGICALLY RESPONSIVE NEOVESSEL' NATURE BIOTECHNOLOGY Bd. 17, November 1999, Seiten 1083 - 1086
- PARNIGOTTO P.P. ET AL: 'EXPERIMENTAL DEFECT IN RABBIT URETHRA REPAIRED WITH ACELLULAR AORTIC MATRIX' UROL. RES. Bd. 28, 2000, SPRINGER-VERLAG, Seiten 46 - 51
- LIPPERT H.: 'Lehrbuch der Anatomie', Bd. 3. AUFL., 1993, URBAN UND SCHWARZENBERG, ISBN 3-541-10063-X Artikel '1 Allgemeine Anatomie, 1.4 Kreislauforgane, # 144', Seiten 39 - 40
- SCHÄFERS H.-J. ET AL: 'Ursachen und Behandlungsstrategien der Aortenklappeninsuffizienz' DEUTSCHES ÄRZTEBLATT Bd. 101, Nr. 37, 10 September 2004, Seiten A2475 - A2479
- SOBOTTA; PUTZ; PABST: 'Atlas der Anatomie des Menschen', Bd. 2, 1993, URBAN UND SCHWARZENBERG, MÜNCHEN, ISBN 3-541-17370-X Artikel 'Rumpf, Eingeweide, untere Extremität', Seite 79

## Beschreibung

Die Erfindung betrifft eine bioartifizielle, primär vaskularisierte Gewebematrix, ein bioartifizielles, primär vaskularisiertes Gewebe, Verfahren zu deren Herstellung sowie deren Verwendung.

Es besteht weltweit ein großer Bedarf an Implantaten und Transplantaten. Die Anzahl an verfügbaren Allotransplantaten, also menschlichen Spenderorganen, ist jedoch begrenzt. Außerdem treten bei der Allotransplantation Abstoßungsreaktionen des Implantats auf, die nur durch eine Immunsuppression oder eine Denaturierung des Gewebes durch Kryokonservierung reduzierbar sind. Dies führt jedoch häufig zu Nebenwirkungen der Immunsuppression oder einem abstoßungsbedingten Verlust des substituierten Gewebes oder Organs. Somit führt die Allotransplantation zwar zu einer Lebensverlängerung bzw. einer Verbesserung der Lebensqualität, jedoch kann mit einem herkömmlichen Allotransplantat das native Gewebe nicht risikolos ersetzt oder dem Patienten die volle Lebensqualität zurückgegeben werden.

Daher wurde in den letzten Jahren intensiv nach alternativen Quellen für Transplantate und Implantate geforscht.

Um die Menge an verfügbaren Transplantaten zu erhöhen, wurde Forschung auf dem Gebiet der Xenotransplantation unter Verwendung tierischen Materials, insbesondere von Schweinen, betrieben. Bei der Xenotransplantation treten jedoch neue Risiken, wie Übertragung von tierischen Krankheiten auf den Menschen, auf.

Eine weitere Möglichkeit, die vorstehend genannten Probleme zu überwinden, ist die Herstellung von bioartifiziellen Geweben, auch tissue engineering genannt. Bei der Herstellung von bioartifiziellen Geweben werden derzeit biologische Matrixsubstanzen, die auf Kollagen basieren, oder biodegradable Kunststoffe als Matrices verwendet. Diese werden sodann mit primären humanen Zellen besiedelt, die aus humanen Biopsien und anschließender Expansion dieser Zellen *in vitro* erhalten werden.

Matrices auf Basis von biodegradablen Kunststoffen haben jedoch den Nachteil, dass unter den verwendeten Zellkulturbedingungen die Umwandlung des Kunststoffgerüsts in funktionelle Bindegewebs-Matrixprotein-Strukturen bzw. deren Neusynthese nicht in ausreichendem Maße möglich ist. Deshalb sind die biochemische Zusammensetzung und die geometrische Struktur von Matrices aus biodegradablen Kunststoffen, die wichtige Differenzierungsfaktoren für das Zellwachstum sind, nicht zufriedenstellend.

Aus diesem Grunde konzentrierten sich viele Forschungsarbeiten in den letzten Jahren auf die Verwendung von biologischen Matrices.

Biologische Matrices können erhalten werden, indem ein geeignetes Gewebe entnommen wird und sodann die darauf befindlichen Zellen durch herkömmliche Azellularisierungsverfahren entfernt werden. Unter anderem fand die *small intestinal submucosa* (SIS) aus dem Schwein in vielen Bereichen des tissue engineering Anwendung, wie beim Ersatz von Blutgefäßen, beispielsweise der Aorta oder der Vena Cava, sowie von Ligamenten, Blase oder Haut.

Allerdings sind biologische Matrices, nachdem diese azellularisiert wurden, instabil und die zum Besiedeln verwendeten Zellen können schon während der Besiedelung nicht ausreichend mit Nährstoffen, Mineralien und/oder Sauerstoff versorgt werden.

Die mit herkömmlichen biologischen Matrices hergestellten bioartifiziellen Gewebe haben den Nachteil, dass diese insbesondere nach der Transplantation nicht ausreichend, beispielsweise mit Nährstoffen, versorgt werden können. Dadurch sterben Zellen des Transplantats ab, der Zell-Matrix-Quotient wird morphologisch verringert, und Zelltod und degenerativer Umbau sind die Folge.

US 5,855,610 offenbart ein Gewebekonstrukt aus Polyglykolsäure, welches vor der Implantation mit Zellen besiedelt wird und um ein Blutgefäß gelegt wird.

WO 99/52356 offenbart ein Gewebekonstrukt aus einem porösen biokompatiblen Material mit aufgetragenen Zellen.

Aufgabe der vorliegenden Erfindung ist es daher, eine bioartifizielle Gewebematrix und ein bioartifizielles Gewebe zur Verfügung zu stellen, die die vorstehend genannten Nachteile von herkömmlichen Matrices und Geweben nicht aufweisen.

Diese Aufgabe wird gelöst durch eine bioartifizielle Gewebematrix nach Anspruch 1 bzw. ein bioartifizielles Gewebe nach Anspruch 10 und durch ein Verfahren zur Herstellung derselben.

Insbesondere wird eine bioartifizielle, primär vaskularisierte Gewebematrix, wenigstens einen funktionsfähigen Gefäßast aufweisend, bereitgestellt. Weiterhin wird ein bioartifizielles, primär vaskularisiertes Gewebe, wenigstens einen funktionsfähigen Gefäßast aufweisend, bereitgestellt.

Als Gewebe zur Herstellung der erfindungsgemäßen Gewebematrix kommen Teile des Magen-Darm-Trakts, insbesondere des Magens, Jejunums, leums oder Colons, in Betracht. Bevorzugt ist das entnommene Gewebe autolog, d.h. es stammt von dem selben Patienten (Spender), dem später das daraus hergestellte Implantat bzw. Transplantat eingesetzt werden soll. Alternativ kommen humane Leichen und tierische Spenderorgane zur Anwendung (Allo/Xenogene Startermatrix).

Das Gewebe ist vorzugsweise mit wenigstens einem vollständigen Gefäßast versehen. Unter Gefäßast wird im Sinne der Erfindung jedes Blutgefäß, wie ein kleinlumiges oder großlumiges Gefäß oder ein Gefäßbaum oder -ast, die mit dem Gewebe verbunden sind, verstanden. Es soll darunter aber auch verstanden werden, dass das primär vaskularisierte Gewebe als Matrix verwendet wird.

Ein solcher Gefäßast besteht vorzugsweise aus einer zuführenden Arterie und einer abführenden Vene. Das Gewebe kann auch mit bis zu 6, vorzugsweise 2 bis 5 Gefäßästen verbunden sein. Besonders bevorzugt ist der Gefäßast mit einem dazugehörigen Netzwerk aus kleinlumigen Gefäßen versehen. Die bevorzugt verwendbare Anzahl an Gefäßästen hängt auch von der Länge und Größe des Implantates ab.

Der Gefäßast oder die Gefäßäste werden bei der Entnahme des Gewebes gesondert präpariert und erhalten, d.h. bei der Isolation des Gewebes wird wenigstens ein funktionsfähiger Gefäßast, der bereits mit dem Gewebe verbunden ist, zusammen mit dem Gewebe entnommen. Der wenigstens eine Gefäßast kann so ausgestaltet sein, dass damit ein intramurales, d.h. alle Wandschichten der Matrix durchziehendes, Kapillarnetz verbunden ist. Der Gefäßast kann ein kleinlumiges Gefäß sein, das mit einem intakten Kapillarnetz verbunden ist, sollte bevorzugt aber so gross sein, dass er im Empfänger (z. B. chirurgisch) an dessen Blutgefäßsystem anzuschliessen (a-nastomosieren) ist.

Wenn das Gewebe ein Darmstück ist, umfaßt der Gefäßast besonders bevorzugt einen arteriellen, größeren Gefäßast und einen großen, venösen Gefäßast, der sich in kleinere Gefäßäste verzweigt. Der Grad der Verzweigung ist abhängig von der Länge des isolierten Darmstücks.

Die Azellularisierung des Gewebes kann mit herkömmlichen Verfahren durchgeführt werden. Im Falle allogenen oder xenogenen Ursprungsmaterials werden Matrix und Gefäßast azellularisiert. Bei Verwendung einer autologen Darmmatrix kann eine Dezellularisierung mit anschliessender autologer Rebesiedlung durch Ko-Kultur folgen. Dadurch kann die Besiedlung des Kapillarnetzes und Gefäßast erhalten bleiben und muß nicht wieder hergestellt werden.

Nach der teilweisen (autolog) oder vollständigen (allogen und xenogen) Azellularisierung wird eine erfindungsgemäße Gewebematrix erhalten. Diese umfasst neben der extrazellulären Matrix des Gewebes selbst außerdem die Matrix des wenigstens einen Gefäßastes. Die erfindungsgemäße Matrix ermöglicht es wegen des Gefäßastes außerdem, beim Besiedeln mit Zellen, auch bei grösseren Zellzahlen oder stärkeren Schichtdicken, diese mit Nährstoffen, Mineralien oder Sauerstoff zu versorgen.

Die erfindungsgemäße Gewebematrix kann sodann mit herkömmlichen Besiedelungsverfahren mit Zellen besiedelt werden.

Die zur Besiedelung verwendeten Zellen können ausgewählt werden aus der Gruppe, bestehend aus autologen, allogenen und xenogenen Zellen. Vorzugsweise sind die Zellen autolog zum späteren Empfänger. Verwendbare Zellen können ausgewählt werden aus der Gruppe, bestehend aus glatten Muskelzellen, Fibroblasten, Endothelzellen, Kardiomyozyten, Chondrozyten, Urethelzellen, Epithelzellen und anderen Zellen.

Mit welchem Zelltyp besiedelt wird, hängt von dem für das jeweilige Einsatzgebiet gewünschten Typ ab, wie Herz, Harnblase oder Luftröhre. Bevorzugt wird mit verschiedenen Zellen besiedelt, die den Zellen entsprechen, die in morphologischen Aufbau und ihrer Funktion dem natürlichen Gewebe entsprechen.

Bei Verwendung einer allogenen oder xenogenen Gewebematrix wird der wenigstens eine Gefäßast vorzugsweise mit Endothelzellen besiedelt. Falls die zur Besiedelung verwendete Gewebematrix autolog bzw. nicht azellulär ist, kann auf eine Besiedelung des Gefäßastes verzichtet werden.

Die zur Besiedelung verwendeten Zellen stammen vorzugsweise aus biotisch gewonnenem Material, insbesondere von dem selben Patienten (Spender), dem später das Transplantat bzw. Implantat eingesetzt werden soll. Die Zellen werden sodann *in vitro* mit herkömmlichen Verfahren expandiert.

Während der Besiedelung können die neu angesiedelten Zellen über den wenigstens einen Gefäßast bereits mit Nährstoffen versorgt werden. Dadurch wird deren Anhaftung an und Einwachsen in die Matrix erleichtert und beschleunigt. Der wenigstens eine Gefäßast wird luminal vorzugsweise mit autologen (empfängereigenen), vaskulären Endothelzellen besiedelt.

Durch das Besiedeln der Gewebematrix mit Zellen wird ein erfindungsgemäßes bioartifizielles, primär vaskularisiertes Gewebe erhalten. Dieses hat den Vorteil, dass es im Gegensatz zu herkömmlichen bioartifiziellen Geweben primär vaskularisiert ist. Zeitgleich mit der Transplantation kann es daher an den Blutkreislauf des Transplantatempfängers angeschlossen werden. Die Zellen in allen Wandschichten des Implantates, also auch in den tiefergelegenen Arealen, können damit mit Nährstoffen versorgt werden und sterben so nicht ab. Damit wird im Gegensatz zu allen bisher verfügbaren bioartifiziellen Implantaten eine frühzeitige Degeneration vermieden und zusätzlich die langfristige Funktion des Transplantats bzw. Implantats sichergestellt.

Wenn der wenigstens eine Gefäßast bereits bei der Entnahme des Gewebes als intakter Gefäßstamm mit angeschlossenen intramuralen Kapillarnetz mit isoliert wird und während der weiteren Verfahrensschritte funktionsfähig gehalten wird, hat das erfindungsgemäße bioartifizielle, primär vaskularisierte Gewebe den Vorteil, bereits während der Herstellung die *in vivo* vorliegende biochemische zelluläre Zusammensetzung und morphologische geometrische Struktur zu besitzen. Damit werden das Einwachsen des Transplantats bzw. Implantats sowie eine den natürlichen Bedingungen und Anforderungen entsprechende Funktionsaufnahme in den Empfänger erleichtert.

Ein weiterer Vorteil des erfindungsgemäßen bioartifiziellen, primär vaskularisierten Gewebes besteht darin, dass die Abstoßungsgefahr durch die Verwendung von autologem, biologischen Material nach der Transplantation minimiert wird.

Das erfindungsgemäße Gewebe kann zur Behandlung jeder Erkrankung eingesetzt werden, die sich durch Transplantation autologer, allogener oder bioartifiziell generierter Gewebe oder Organe behandeln lassen.

Besonders bevorzugte Einsatzgebiete sind bioartifizielle links- oder rechtsventrikuläre Herz-Unterstützungs- oder Komplettsysteme, kardiale, vaskularisierte Patchplastiken zur Therapie von narbigen Folgen des Herzinfarkts, aneurysmatischer Aussackungen oder angeborener Fehlbildungen mit fehlender Anlage eines Herzanteiles, wie hypoplastischer rechter Ventrikel, Ersatz für Blutgefäße, beispielsweise für Aortenersatz Bypassoperationen, vaskularisierter Ersatz im Bereich des gesamten Gastrointestinal-Trakts bei hereditären, entzündlichen, tumorösen, vaskulären, degenerativen oder verletzungsbedingten Erkrankungen des Schlundes, der Speiseröhre, des Magens sowie des Dünn- und Dickdarms, vaskularisierter Bindegewebersatz, geeignet auch für hohe dynamische Beanspruchungen, wie Leistenhernie, Lungen-, Trachea- und Bronchialersatz bei pulmonalen und trachealen Fehlanlagen oder Erkrankungen, gestielter Hautersatz zur Behandlung von Hautdefekten, wie bei großflächigen Brandverletzungen, vaskularisierter Ersatz von Geweben und Organen im Urogenitalbereich, wie Niere, Harnleiter, Blase oder Harnröhre, z.B. bei Morbus Ormond Fehlanlagen oder Verletzungen, und als vaskularisierter Ersatz von Knochengeweben bei Fehlanlage, nach Verlust durch Trauma, Tumor oder Entzündungen.

## Patentansprüche

1. Verfahren zur Herstellung einer bioartifiziellen, primär vaskularisierten autologen, allogenen oder xenogenen Gewebematrix, die Schritte umfassend
Bereitstellen eines entnommenen autologen, allogenen oder xenogenen Gewebes mit wenigstens einem damit verbundenen funktionsfähigen Gefäßast, wobei das Gewebe aus dem Magen-Darm-Trakt stammt, und
Azellularisieren des bereitgestellten Gewebes unter Erhaltung der Funktionsfähigkeit des wenigstens einen Gefäßastes,
wobei das entnommene Gewebe autolog ist und der wenigstens eine Gefäßast nicht azellularisiert wird oder
wobei das entnommene Gewebe allogen oder xenogen ist und der wenigstens eine Gefäßast vollständig azellularisiert wird.

2. Verfahren nach Anspruch 1, in welchem die Gewebematrix wenigstens zwei Matrices von Gefäßästen umfasst.

3. Verfahren nach Anspruch 2, in welchem die wenigstens zwei Matrices von Gefäßästen wenigstens eine Matrix eines arteriellen und wenigstens eine Matrix eines venösen Gefäßasts sind.

4. Verfahren nach einem der Ansprüche 1 bis 3, in welchem die Matrix des wenigstens einen Gefäßasts mit einem intramuralen Kapillametz, das in der Matrix vorhanden ist, verbunden ist.

5. Verfahren nach einem der Ansprüche 1 bis 4, in welchem die Matrix des wenigstens einen Gefäßastes die Matrix eines Gefäßbaumes ist.

6. Verfahren nach einem der Ansprüche 1 bis 5, in welchem die Matrix des wenigstens einen Gefäßasts die Matrix eines kleinlumigen Gefäßasts ist.

7. Verfahren zur Herstellung eines bioartifiziellen, vaskularisierten Gewebes, umfassend die Schritte
Herstellen einer Gewebematrix gemaß dem Verfahren nach einem der Ansprüche 1 bis 6 und
Besiedeln der Gewebematrix mit autologen, allogenen und/oder xenogenen Zellen.

8. Verfahren nach Anspruch 7, in welchen die Gewebematrix mit autologen Zellen besiedelt wird.

9. Verfahren nach Anspruch 7 oder 8, in welchem während der Besiedelung die zum Besiedeln verwendeten Zellen über den Gefäßast mit Nährstoffen versorgt werden.

10. Bioartifizielle, primär vaskularisierte Gewebematrix/bioartifizielles, primär vaskularisiertes Gewebe, erhältlich durch ein Verfahren nach einem der Ansprüche 1 bis 9.

## Claims

1. Method for producing a bioartificial, primarily vascularized autologous, allogenic, or xenogenic tissue matrix, comprising the following steps:
providing a removed autologous, allogenic, or xenogenic tissue with at least one functional vessel branch connected thereto, the tissue originating from the gastrointestinal tract, and
acellularizing the provided tissue while preserving the functionality of the least one vessel branch,
wherein the removed tissue is autologous and the at least one vessel branch is not acellularized, or
wherein the removed tissue is allogenic or xenogenic and the at least one vessel branch is completely acellularized.

2. Method according to Claim 1, wherein the tissue matrix includes at least two matrices of vessel branches.

3. Method according to Claim 2, wherein the at least two matrices of vessel branches represent at least one matrix of an arterial vessel branch and at least one matrix of a venous vessel branch.

4. Method according to one of Claims 1 through 3, wherein the matrix of the at least one vessel branch is connected to an intramural capillary network which is present
in the matrix.

5. Method according to one of Claims 1 through 4, wherein the matrix of the at least one vessel branch is the matrix of a vessel tree.

6. Method according to one of Claims 1 through 5, wherein the matrix of the at least one vessel branch is the matrix of a small-lumen vessel branch.

7. Method for producing a bioartificial, vascularized tissue, comprising the following steps:
providing a tissue matrix using the method according to one of Claims 1 through 6, and
populating the tissue matrix with autologous, allogenic, and/or xenogenic cells.

8. Method according to Claim 7, wherein the tissue matrix is populated with autologous cells.

9. Method according to Claim 7 or 8, wherein during the population, the cells used for populating are supplied with nutrients via the vessel branch.

10. Bioartificial, primarily vascularized tissue matrix/bioartificial, primarily vascularized tissue, obtainable using a method according to one of Claims 1 through 9.

## Revendications

1. Procédé de fabrication d'une matrice tissulaire bioartificielle autologue, allogène ou xénogène, à vascularisation primaire, comprenant les étapes de :
préparation d'un tissu prélevé autologue, allogène ou xénogène, avec au moins une branche vasculaire fonctionnelle qui lui est associée, ledit tissu provenant du système gastro-intestinal, et
acellularisation du tissu préparé, sous respect de la fonctionnalité de la au moins une branche vasculaire,
le tissu prélevé étant autologue et la ou les branches vasculaires n'étant pas acellularisées, ou bien
le tissu prélevé étant allogène ou xénogène et la ou les branches vasculaires étant complètement acellularisées.

2. Procédé selon la revendication 1, dans lequel la matrice tissulaire comprend au moins deux matrices de branches vasculaires.

3. Procédé selon la revendication 2, dans lequel les deux matrices de branches vasculaires au moins sont au moins une matrice d'une branche vasculaire artérielle et au moins une matrice d'une branche vasculaire veineuse.

4. Procédé selon l'une des revendications 1 à 3, dans lequel la matrice de la ou des branches artérielles est reliée à un réseau capillaire intramural, qui est présent dans la matrice.

5. Procédé selon l'une des revendications 1 à 4, dans lequel la matrice de la ou des branches vasculaires est la matrice d'un arbre vasculaire.

6. Procédé selon l'une des revendications 1 à 5, dans lequel la matrice de la ou des branches vasculaires est la matrice d'une branche vasculaire à petit lumen.

7. Procédé de fabrication d'un tissu bioartificiel vascularisé, comprenant les étapes de :
fabrication d'une matrice tissulaire selon l'une des revendications 1 à 6, et
colonisation de la matrice tissulaire avec des cellules autologues, allogènes et/ou xénogènes.

8. Procédé selon la revendication 7, dans lequel la matrice tissulaire est colonisée avec des cellules autologues.

9. Procédé selon la revendication 7 ou 8, dans lequel, par l'intermédiaire de la branche vasculaire, les cellules utilisées pour la colonisation sont alimentées en nutriments pendant la colonisation.

10. Matrice tissulaire bioartificielle, à vascularisation primaire/tissu bioartificiel, à vascularisation primaire, susceptible d'être obtenu par un procédé selon l'une des revendications 1 à 9.
